# EUROPEAN PATENT APPLICATION

(11) **EP 4 070 856 A1**
(43) Date of publication of application: **12.10.2022**
(21) Application number: 21167366.0
(22) Date of filing: 08.04.2021
(51) Int. Cl.: A61P 33/06, A61K 31/16, C07C 259/18

(54) **ORGANIC COMPOUNDS AND USES THEREOF**

(71) Applicant: Adebiyi, Ezekiel, Ota (NG); Ajani, Olayinka, Ota (NG); Klika, Karel, 69129 Heidelberg (DE)
(72) Inventor: Adebiyi, Ezekiel, Ota (NG); Ajani, Olayinka, Ota (NG); Klika, Karel, 69129 Heidelberg (DE)
(74) Representative: Ullrich & Naumann PartG mbB

(57) **Abstract**

The present invention relates to compounds and in particular to organic compounds useful in the treatment of malaria. The invention further relates to uses of these organic compounds and embodiments of the invention have been particularly developed for uses of the compounds of the invention in medical treatment as well as in *in vitro* laboratory research and will be described hereinafter with reference to these applications.

## Description

### FIELD OF THE INVENTION

The present invention relates to compounds and in particular to organic compounds useful in the treatment of malaria. The invention further relates to uses of these organic compounds and embodiments of the invention have been particularly developed for uses of the compounds of the invention in medical treatment as well as in in vitro laboratory research and will be described hereinafter with reference to these applications. However, it will be appreciated that the invention is not limited to this particular field of use.

### BACKGROUND OF THE INVENTION

Any discussion of the background art throughout the specification should in no way be considered as an admission that such art is widely known or forms part of common general knowledge in the field.

According to the World Health Organisation (WHO), 228 million cases of malaria occurred worldwide with the most cases reported in African Region. Furthermore, children under the age of five are the most vulnerable group affected by malaria as they account for 67% of all malaria deaths worldwide. There are five species of the *Plasmodium* parasite that cause malaria in humans, with *Plasmodium falciparum* and *Plasmodium viva* being the most prevalent in Africa and countries outside Africa, respectively. Despite continuing efforts to eradicate the malaria-causing pathogens, there is an increase in pathogen resistance to the most effective drugs such as against artemisinin, mefloquine and piperaquine in South East Asian countries of Thailand and Cambodia.

Accordingly, the need for additional and/or improved anti-infective agents, in particular for anti-infective agents effective in preventing and or treating infectious diseases caused by *Plasmodium sp.* such as malaria, still exists in the art.

It is an object of the present invention to overcome or ameliorate at least one of the disadvantages of the prior art, or to provide a useful alternative. In particular, it is an object of the present invention to provide novel compounds for medical treatment of infectious diseases such as compounds for improved malaria therapy.

### SUMMARY OF THE INVENTION

The present invention provides a solution to the above-formulated problem based on the inventors' surprising finding that certain amidinyl-, guanidinyl- and amidoximyl based derivatives, in particular compounds of *Formula A,* are capable of disrupting folate synthesis of infectious pathogens such as, for example, the folate synthesis pathway of *Plasmodium sp.*

The compounds of the present invention have anti-plasmodial activity, which makes them particularly useful as anti-malarial agents in the treatment of malaria, but also have the capacity to act as anti-infective agents due to their capacity to bind to and enzymatically inhibit 6-pyruvoyl tetrahydropterin synthase (PTPS). In the context of malaria, the compounds of the present invention are particularly useful due to their capacity to bind to and enzymatically inhibit the *Plasmodium falciparum* PTPS (P*F*PTPS), PFF1360w.

The compounds of the present invention have been identified in a structure-based *in silico* drug discovery approach and have promising antimalarial activity. In particular, molecular modelling techniques such as QSAR (Quantitative Structure Activity Relationships) and molecular docking where applied to identify regions and spaces within the tertiary structure of PTPS that are considered convey and/or effect the PTPS' enzymatic activity. The binding affinities of organic compounds that can occupy such regions and spaces can be calculated such that the ability of a compound to interfere with the biological activity of the biomolecule can be predicted. It has therefore been possible to design and develop bioactive compounds as targeted inhibitors of PTPS and in particular to *Plasmodium falciparum* PTPS, namely of PFF1360w. The compounds identified in this approach have been synthesized *de novo.*

Accordingly, in a first aspect, the present invention relates to a compound selected from group consisting of:
a compound of *Formula A* 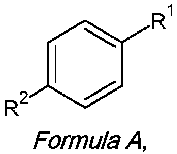 wherein R¹ is 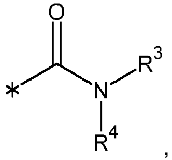 wherein R³ is hydroxy or C₁-C₄ alkoxy and R⁴ is hydrogen, or
wherein R¹ is 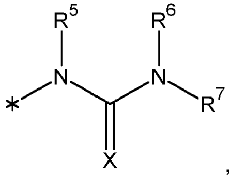 wherein R⁵ and R⁶ are hydrogen, R⁷ is hydroxy or benzyloxy, and X is O or S, or
wherein R¹ is 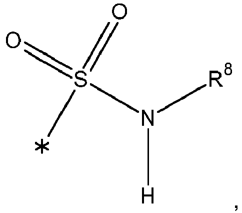 wherein R⁸ is hydroxy, and
wherein R² is selected from the group consisting of: 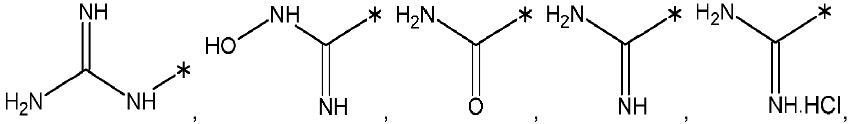 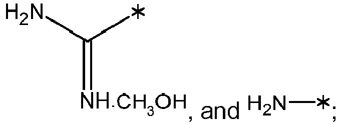 and
wherein * denotes the junction of the indicated bond with an atom of the compound of *Formula A;*

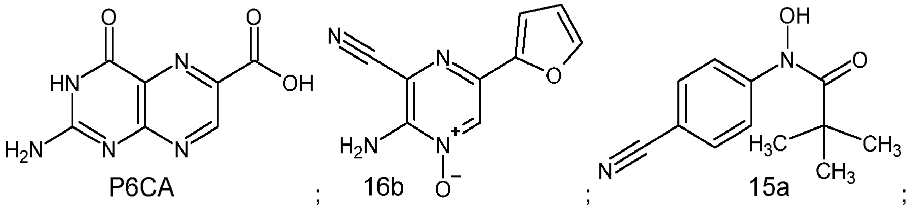

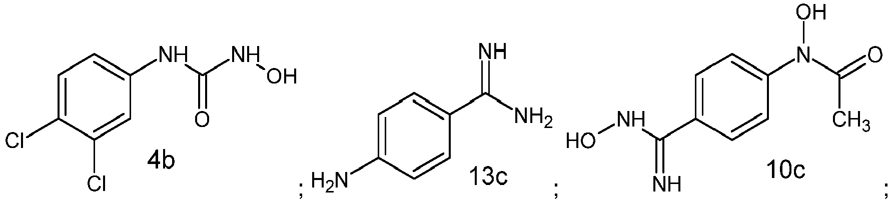

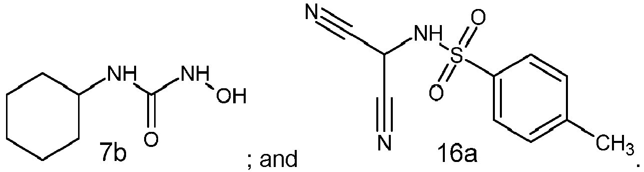

Preferably, the compound of *Formula A* is 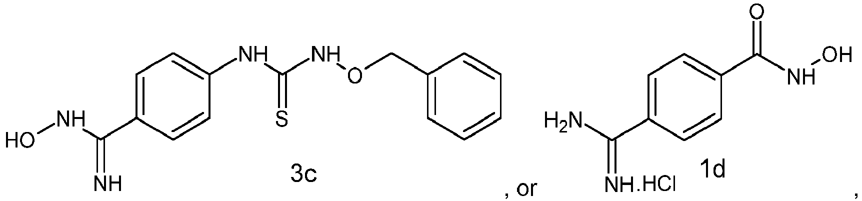 or

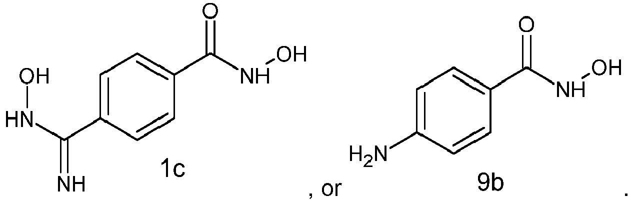

An embodiment of the present invention relates to the compound of the first aspect for use as a medicament. For example, to the compound of the first aspect for use in the treatment of an infectious disease, to the compound of the first aspect for use in the treatment of malaria.

As such, in further aspects, the present invention also relates to a method of medical treatment such as the treatment of an infectious disease and/or the treatment of malaria, said method comprising administering a compound of the first aspect to a patient in need thereof.

Similarly, in a further aspect, the present invention also relates to use of a compound of the first aspect in the manufacture of a medicament product for medical treatment such as for the treatment of an infectious disease and/or for the treatment of malaria.

In yet a further aspect, the present invention relates to use of the compound of the first aspect for disrupting folate synthesis in *Plasmodium sp.,* preferably in *Plasmodium falciparum.*

In yet a further aspect, the present invention relates to use of a compound of the first aspect for inhibiting 6-pyruvoyltetrahydropterin synthase (PTPS) in *Plasmodium sp..* Preferably, the 6-pyruvoyltetrahydropterin synthase is *Plasmodium falciparum* 6-pyruvoyltetrahydropterin synthase (P*F*PTPS), in particular PFF1360w.

### FIGURES

Embodiments of the invention will now be described, by way of example only, with reference to the accompanying drawings in which:
- Fig. 1: shows an IC₅₀ curve for chloroquine determined in the *P. falciparum* NF54 strain.

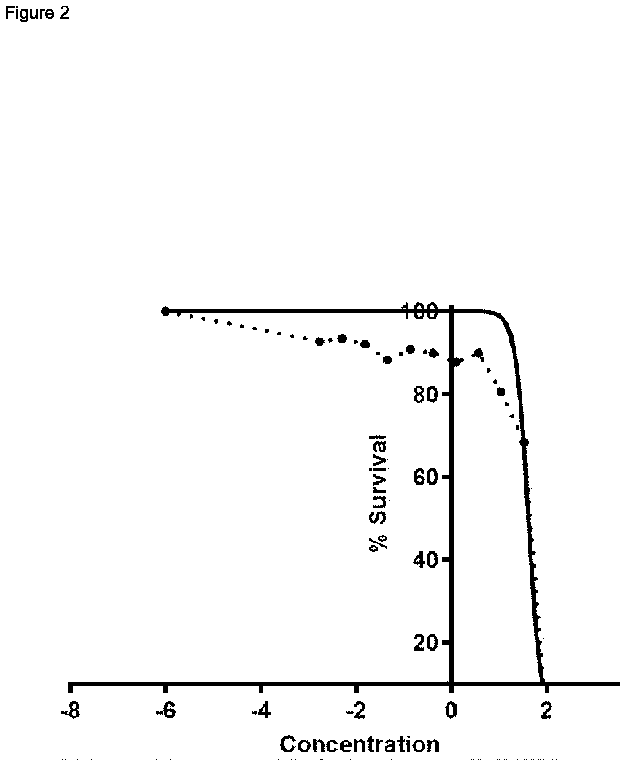

- Fig. 2: shows an IC₅₀ curve for Compound 1d in the NF54 strain.

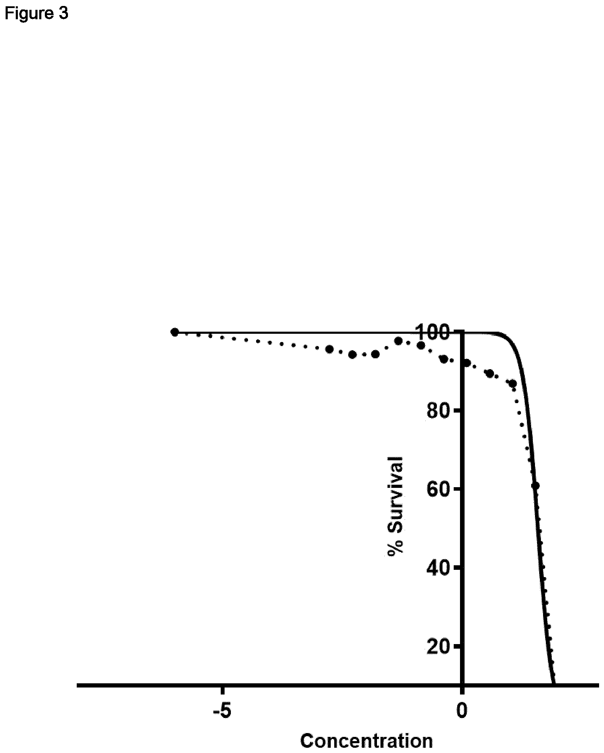

- Fig. 3: shows an IC₅₀ curve for Compound 9d in the NF54 strain.

### DETAILED DESCRIPTION OF THE INVENTION

More and more drug design pipelines for the identification and development of novel anti-infective agents are based on research methods that involve predicting the activities of new compounds before the compounds are indeed synthesized. This has resulted in the use of molecular modelling techniques such as QSAR (Quantitative Structure Activity Relationships) and molecular docking, which help to identify regions and spaces within a biomolecule's structure, e.g. within a protein's tertiary structure, that are thought to convey and/or effect the biomolecule's biological activity. The binding affinities of organic compounds that can occupy such regions and spaces can be calculated such that the ability of a compound to interfere with the biological activity of the biomolecule can be predicted. It is therefore possible to design and develop bioactive compounds as targeted inhibitors of biomolecules. The compounds of the present invention have been identified in such a structure-based *in silico* drug discovery approach and have been shown to have promising antimalarial activity as predicted by the binding affinities of these compounds to 6-pyruvoyl tetrahydropterin synthase (PTPS), *Plasmodium falciparum* PTPS (*PF*PTPS) and PFF1360w, calculated prior to the compounds' synthesis.

As already indicated, the compounds of the present invention have anti-plasmodial activity, which makes them particularly useful as anti-malarial agents in the treatment of malaria, but also have the capacity to act as anti-infective agents generally due to their capacity to bind to and enzymatically inhibit 6-pyruvoyl tetrahydropterin synthase (PTPS). In addition, the compounds may further bind to and inhibit closely related proteins having similar tertiary structures and enzymatic pockets/activities such as to provide an alternative and/or dual target mechanism. In the context of malaria, this is considered particularly beneficial as it firstly reduces the likelihood of drug resistance developing in infective species of *Plasmodium* but also provides a mechanism of action potentially suitable for the same compound to target differing species of *Plasmodium.* The compounds of the present invention are particularly useful due to their capacity to bind to and enzymatically inhibit the *Plasmodium falciparum* PTPS (*PF*PTPS), PFF1360w.

In order to provide a clear and consistent understanding of the specification and claims, and the scope to be given such terms, the following definitions are provided.

### Definitions

In the context of the present disclosure, **"hydroxy"** or **"hydroxyl group"** means the entity with the chemical formula OH.

In the context of the present disclosure, **"C₁-C₄ alkoxy"** *means* an alkyl-group comprising 1 to 4 carbon atoms, namely a methyl-, ethyl-, propyl- or butyl-group, singularly bonded to oxygen. Similarly, a **"benzyloxy"** is a benzyl group singularly bonded to oxygen.
In the context of the present disclosure, in particular in the context of the chemical compound structures shown, "*" denotes the junction of the indicated bond with an atom of the compound shown.

In addition to the above definitions, and unless the context clearly requires otherwise, throughout the description and the claims, the words **"comprise", "comprising",** and the like are to be construed in an inclusive sense as opposed to an exclusive or exhaustive sense; that is to say, in the sense of "including, but not limited to".

Further, reference throughout this specification to **"one embodiment", "some embodiments"** or **"an embodiment"** means that a particular feature, structure or characteristic described in connection with the embodiment is included in at least one embodiment of the present invention. Thus, appearances of the phrases "in one embodiment", "in some embodiments" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

As used herein, unless otherwise specified the use of the ordinal adjectives **"first", "second", "third",** etc., to describe a common object, merely indicate that different instances of like objects are being referred to, and are not intended to imply that the objects so described must be in a given sequence, either temporally, spatially, in ranking, or in any other manner.

When used herein, the term **"exemplary"** is used in the sense of providing examples, as opposed to indicating quality. That is, an **"exemplary embodiment"** is an embodiment provided as an example, as opposed to necessarily being an embodiment of exemplary quality.

### ¹H-NMR and 13C-NMR spectra

The ¹H-NMR and ¹³C-NMR spectra were recorded in DMSO-d₆ on JEOL Delta NMR ECX400 spectrometer operating at 400 MHz and 100 MHz respectively. The deuterium signal of the solvent was the lock and chemical shifts δ was recorded in ppm. The Mass Spectra were obtained using Q-Trap 2000 Spectrometer at 70 eV. The elemental analysis (C, H, N) of the compounds were performed using various MICRO V1.5.1 Elemental Analyzer. The reaction progress was monitored with TLC using CHCl₃/CH₃OH (99%, 95%) solvent system and the developed plates were visualized under UV lamp where necessary. Distortionless Enhancement by Polarization Transfer (DEPT-135) test was carried out to identify proton-attached carbon atoms of methyl, methylene and methane. Column chromatographic purifications were carried out on Merck silica gel F (Mesh 200-300) using DCM / MeOH as eluting solvent, but in varied ratio depending on the polarity of the crude compounds. Organic solutions were dried over anhydrous MgSO₄ while concentration and removal of solvents was achieved with RE-2000B Rotary Evaporator at reduced pressure. All chemicals were obtained from Sigma-Aldrich Chemicals and other manufacturers in Germany.

### Antiplasmodial Screening Procedure

*In vitro* activity against erythrocytic stages of *P. falciparum* was determined using a ³H-hypoxanthine incorporation assay using the drug sensitive NF54 strain and the standard drugs chloroquine (Sigma C6628). Compounds were dissolved in DMSO at 10 mg/ml and further diluted in medium before they were added to parasite cultures incubated in RPMI 1640 medium without hypoxanthine, supplemented with HEPES (5.94 g/l), NaHCO₃ (2.1 g/l), neomycin (100 U/ml), Albumax^{R} (5 g/l) and washed human red cells A⁺ at 2.5% haematocrit (0.3% parasitaemia). Serial drug dilutions of seven 2-fold dilution steps covering a range from 10 to 0.02 µg/ml were prepared. The 96-well plates were incubated in a humidified atmosphere at 37 °C; 4% CO₂, 3% O₂, 93% N₂. After 48 h 50 µl of ³H-hypoxanthine (=0.5 µCi) was added to each well of the plate. The plates were incubated for a further 24 h under the same conditions. They were then harvested with a Betaplate^{™} cell harvester (Wallac, Zurich, Switzerland), and the red blood cells transferred onto a glass fibre filter then washed with distilled water. The dried filters were inserted into a plastic foil with 10 ml of scintillation fluid, and counted in a Betaplate^{™} liquid scintillation counter (Wallac, Zurich, Switzerland). IC₅₀ values were calculated from sigmoidal inhibition curves by linear regression using Microsoft Excel. Chloroquine and artemisinin were used as control.

### In vitro cytotoxicity with L-6 cells.

Assays were performed in 96-well microtiter plates, each well containing 100 µl of RPMI 1640 medium supplemented with 1% L-glutamine (200mM) and 10% fetal bovine serum, and 4000 L-6 cells (a primary cell line derived from rat skeletal myoblasts) [15]. Serial drug dilutions of eleven 3-fold dilution steps covering a range from 100 to 0.002 µg/ml were prepared. After 70hours of incubation the plates were inspected under an inverted microscope to assure growth of the controls and sterile conditions. 10µl of Alamar Blue was then added to each well and the plates incubated for another 2 hours. Later they were read with a Spectramax Gemini XS microplate fluorometer (Molecular Devices Cooperation, Sunnyvale, CA, USA) using an excitation wave length of 536 nm and an emission wave length of 588 nm. The IC50 values were calculated by linear regression [16] from the sigmoidal dose inhibition curves using SoftmaxPro software (Molecular Devices Cooperation, Sunnyvale, CA, USA). Podophyllotoxin (Sigma P4405) as control.

### Molecular Docking

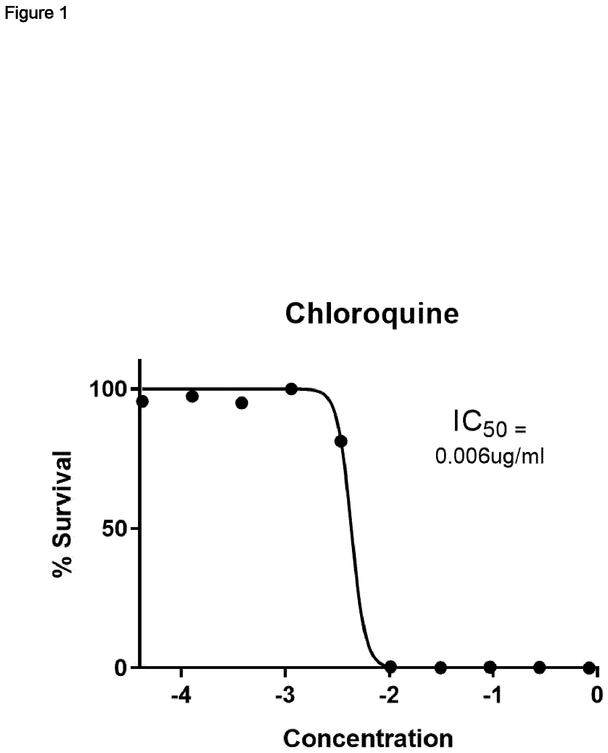

For docking studies, the *P. falciparum* 6-pyruvoyltetrahydropterin synthase (*Pf*PTPS) crystal structure with the PDB code 1Y13 (https://www.ebi.ac.uk/pdbe/entry/pdb/1Y13) was used. 1Y13 has a resolution of 2.2Å. The receptor was defined by a radius of 8.0 Å around the native ligand in the binding pocket. The active site of *Pf*PTPS is rather small and contains a zinc ion. Due to the size of the binding pocket and the zinc ion in the active site, the fragment-like subset of the ZINC Database was screened using a graph-matching strategy for zinc-binding groups. Also, the negative charged glutamate and aromatic side chains in the identified binding pocket explain the binding mode of the native binder biopterin. In the deeper pocket, a negative charged glutamate and aromatic side chains explain the binding mode of the native binder, biopterin. The complexed native binder, biopterin was removed from the protein structure using Chimera software. Furthermore, the protein was prepared by the removal of non-amino acid residues and water molecules, computation of Gasteiger charges, addition of polar hydrogens and merging of the nonpolar hydrogens using AutoDockTools 1.5.6. The 2-dimensional (2D) structures of the designed compounds (Fig. 1), biopterin ligand and chloroquine, were built using ChemDraw Professional 15.0 by PerkinElmer USA. Also, ChemDraw was used to generate the simplified molecular-input line-entry system (SMILES) that were converted to their corresponding 3D structures using FRee Online druG 3D conformation generator (FROG) and saved in .mol2 format. In addition, OpenBabel software was used to convert the .mol2 files to the AutoDock docking format (.pdbqt), which was further used for the docking simulation. The grid box was constructed using 60, 60, and 60, pointing in x, y, and z directions, respectively, with a grid point spacing of 0.375 Å. The centre grid box is of 88.335 Å, 35.930 Å and 21.447 Å around Glu B128, Thr B127, His B29, Glu B161, Glu B38, His B43, Zn 174, Ile B31, Phe A64, Ile A62, TyrA60 in the binding pocket. The molecular docking analysis was performed carried out using Autodock vina and the post docking analysis was carried out using PyMol.

Specifically, and to further illustrate, from 413,796 entries on ZINC Database, 12,068 compounds contained at least one zinc-binding group. The number of unique compounds was 4,140. These unique compounds were *in silico* docked in the binding pocket using the GOLD program (CCDC, UK) and we chose the ASP scoring function to evaluate interactions of predicted poses. Five possible docking solutions for each compound were clustered at a RMSD of 2.0Å. All solutions were re-scored by Drugscore CSD and ranked by the per-atom-score. The top 200 solutions were inspected visually. The selected ligands were optimized and a basic functionality has been added in order to enable the interaction to a negatively charged glutamate 128 at the bottom of the binding pocket. The screening results were also modified in regard to more simple synthetic approach such as to arrive at the compounds of the present invention.

All compounds of the present invention have either one of the following zinc-binding groups (sulfonamide-fused, urea-fused, amide-fused) shown to enable the binding of the zinc ion in the binding pocket of the active site of *Pf*PTPS. Also, there is the incorporation of the amidine/amidoxime fused templates, which can be a guanidine-based template. Without wanting to be bound by theory, the amidoxime side chain functionality may prove to be an essential feature due to its role in prodrug conversion because amidoxime, when orally administered has been known to undergo reduction within the human body such as to act in its amidine form delivering better compound efficacy.

Zinc-binding groups that were incorporated in the structure of the compounds of the invention, based on the ZINC Database fragment-like subset of the docking experiment:

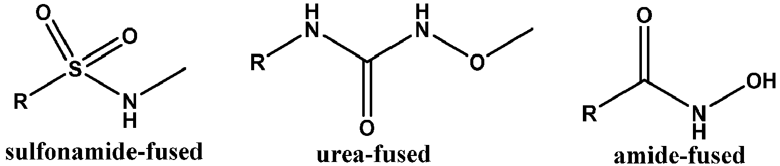

The incorporated guanidinyl-based, amidinyl-based, amidoximyl-based groups:

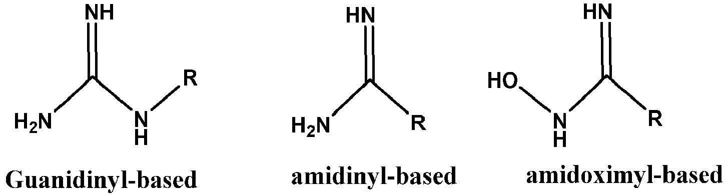

### RESULTS AND DISCUSSION

### Antimalarial activity and in vitro cytotoxicity

The following are the preferred compounds of the present invention:

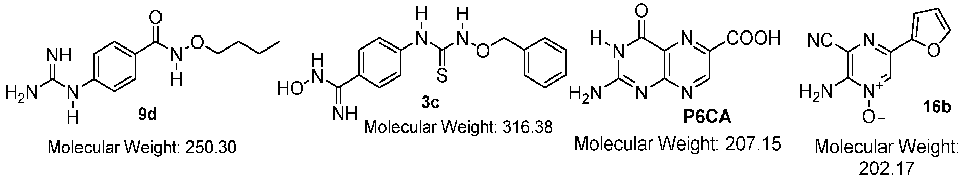

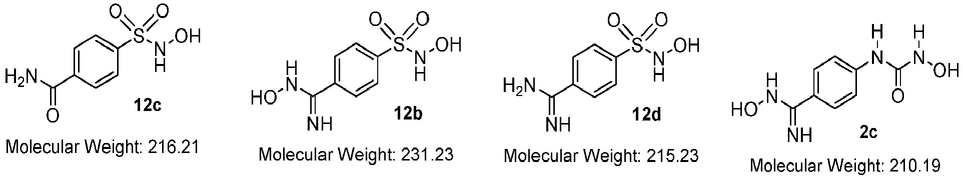

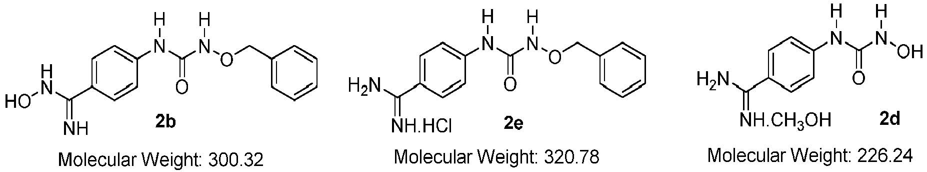

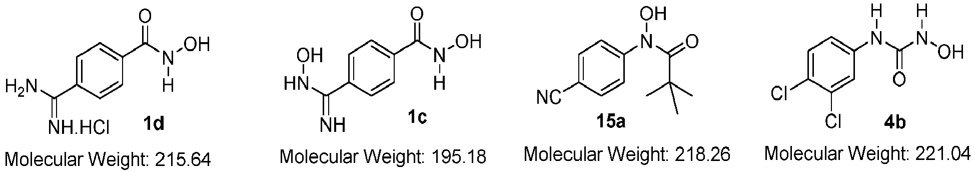

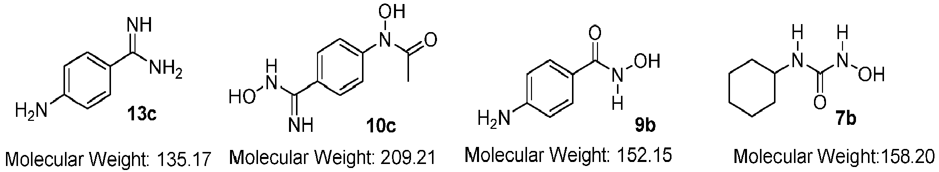

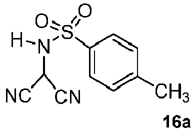

As will be appreciated the above-shown 20 preferred compounds are amidoximyl-fused (5), amidinyl-fused (4), amide-fused (2), sulfonamide-fused (3), urea-fused (3), guanidinyl-based (1), cyanido-fused (2) compounds.

These preferred compounds were tested *in vitro* antimalarial activity against the drug sensitive NF54 strain of *P. falciparum* and the standard drugs chloroquine (Sigma C6628) using a ³H-hypoxanthine incorporation assay. Antimalarial activity (µg/ml) was quantified as inhibition of parasite growth and results are presented in Table 1.

Not all the screened compounds were as active as the standard drug chloroquine, however, four of the compounds have IC₅₀ values still within accepted ranges i.e. IC₅₀ values lower that 10 µg/ml. Compounds **1c, 1d, 3c** and **9b** are the most potent among screened compounds based the antimalarial screening.

The cytotoxicity activities on L6 cell also showed that the screened compounds had moderate cytotoxicity to non-cytotoxicity when compared to the standard cytotoxicity compound (podophyllotoxin) with the IC₅₀ values greater than 33.55 µg/ml. Importantly, although, chloroquine has a lower IC₅₀ than the compounds of the present invention, its mechanism of action with respect to inhibiting *Plasmodium* is significantly different from that of the compounds of the present invention. Specifically, within *Plasmodium,* chloroquine acts by inhibiting the conversion of hemozoin from heme, thereby leading to the accumulation of toxic heme within the parasite. However, free heme is not only toxic to the parasite but also to cells in general, causing undesirable side effects in the patient being treated.

In contrast, the new compounds act through the inhibition of *P. falciparum* 6-pyruvoyltetrahydropterin synthase without the generation of free, toxic heme. Also, the lower IC₅₀ values obtained or some of the compounds is also encouraging because of some of them only have moderate toxicity (generally classified to be between 2-89 µg/ml), while others had no toxicity at all (>100µg/ml) as compared to standard cytotoxic agent used, podophyllotoxin (IC₅₀ = 0.006 µg/ml).

**Table 1: Results of in vitro antimalarial activities and cytotoxicity activities of desired synthesized compounds**

| **Compound ID** | *P. falciparum* NF54 IC₅₀ (µg/ml) | Cytotoxicity L6 IC₅₀ (µg/ml) |
|---|---|---|
| 1c | 2.075 | 56.1 |
| 1d | 0.923 | 91.1 |
| 2b | >10 | 43 |
| 2c | >10 | >100 |
| 2d | >10 | >100 |
| 2e | >10 | 75 |
| 3c | 7.6 | 57.65 |
| 4b | >10 | >100 |
| 7b | >10 | 93 |
| 9b | 0.967 | 43.4 |
| 9d | >10 | >100 |
| 10c | >10 | 52.2 |
| 12b | >10 | >100 |
| 12c | >10 | >100 |
| 12d | >10 | >100 |
| 13c | >10 | 42.85 |
| 15a | >10 | 33.55 |
| 16a | >10 | >100 |
| 16b | >10 | 61.15 |
| P6CAH | >10 | >100 |
| Chloroquine | 0.004 | |
| Podophyllotoxin | | 0.006 |

### Molecular Docking

The molecular docking studies of all the 20 screened compounds in the binding pocket of *P. falciparum* 6-pyruvoyltetrahydropterin synthase (*Pf*PTPS) showed that their binding affinities ranged between -6.9 and -5.2 kcal/mol in the order: 12d > 2e > 9d > 2b > P6CAH > 2d > 2c > 12c > 1c > 7b > 10c > 12b > 13c > 15a > 16b > 16a > 1d > 4b > 3c > 9b. The native ligand, biopterin had a binding affinity of -6.1 kcal/mol and chloroquine -5.5 kcal/mol. The four compounds with the highest 1C₅₀ values from the antimalarial screening, **1c, 1d, 3c** and **9b** have binding affinity of -6.0, -5.4, -5.2 and -5.2 respectively. Even though these compounds are not the best binders amongst the 20 screened compounds against the *Pf*PTPS, their binding affinities still suggest that they are good inhibitors of the target protein.

**Table 2: The binding affinities of the 20 screened compounds, biopterin and chloroquine in the binding pocket of 1Y13.**

| **Compound ID** | **Binding affinity (against 1Y13) (kcal/mol)** |
|---|---|
| 1c | -6.0 |
| 1d | -5.4 |
| 2b | -6.5 |
| 2c | -6.2 |
| 2d | -6.3 |
| 2e | -6.8 |
| 3c | -5.2 |
| 4b | -5.4 |
| 7b | -5.9 |
| 9b | -5.2 |
| 9d | -6.7 |
| 10c | -5.9 |
| 12b | -5.8 |
| 12c | -6.2 |
| 12d | -6.9 |
| 13c | -5.8 |
| 15a | -5.8 |
| 16a | -5.6 |
| 16b | -5.8 |
| P6CAH | -6.5 |
| Chloroquine | -5.5 |
| Biopterin | -6.1 |

### Conclusion

All living organisms require folate cofactors as essential molecules. *Plasmodium falciparum*n is capable of both *de novo* folate biosynthesis and salvage of pre-formed folate from the plasma of its human host. Research shows that both routes are essential for continued healthy parasite growth and the inhibition of reactions in folate metabolism has been the basis of combinatorial advances (such as pyrimitamine and sulfadoxine) against malaria. However, the use of some of the combinatorial drugs has an increased resistance rate. This study was taken to assess the antimalarial activity of amidinyl-, guanidinyl- and amidoximyl based on derivatives targeting the *P. falciparum* 6-pyruvoyltetrahydropterin synthase (*Pf*PTPS), disturbing the folate synthesis pathway of *P.falciparum.* The *in silico* docking was correlated with the *in vitro* antimalarial screening in order to develop the compounds of the present invention having good anti-plasmodial activity. The high binding energy of the synthesized derivatives showed that the designed analogues have good affinities for the PFF1360w protein, which is likely to be the reason for its significant antimalarial activity against *P. falciparum.*

Again, it is important to appreciate that the present invention, namely the newly synthesized compounds/drugs of the present invention, is the result of a structure-based virtual screening procedure with a clear protein target in mind (PFF1630w), which was identified as a potential antimalarial drug target, disturbing the folate synthesis pathway of *Plasmodium falciparum.*

Accordingly, on one hand, the compounds of the present invention are particularly suitable for use as a medicament per se and in particular for use in the treatment of an infectious disease, such as, e.g. malaria.

On the other hand, the compounds of the present invention are useful in a non-medical setting such as in the field of basic laboratory research into malaria and can serve as useful research tools to be employed in various screens and assays. For example, the compounds of the present invention can be used particularly well for disrupting folate synthesis in *Plasmodium sp.,* in particular in *P. falciparum.* Typically, the compounds of the invention can be used for inhibiting 6-pyruvoyltetrahydropterin synthase (PTPS) in *Plasmodium sp..* For example in *Plasmodium falciparum* through inhibiting the *Plasmodium falciparum* 6-pyruvoyltetrahydropterin synthase (PFPTPS). Typically, the PFPTPS is PFF1360w. The use of the compounds of the present invention harbour great promise both with respect to the advancement of malaria therapy directly but also as tools in further malaria research.

Many modifications and other embodiments of the invention set forth herein will come to mind to the one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. A compound selected from group consisting of:
a compound of ***Formula A*** 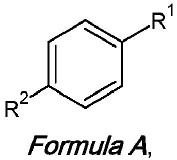 wherein R¹ is 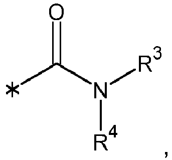 wherein R³ is hydroxy or C₁-C₄ alkoxy and R⁴ is hydrogen, or wherein R¹ is 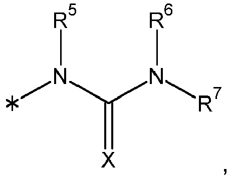 wherein R⁵ and R⁶ are hydrogen, R⁷ is hydroxy or benzyloxy, and X is O or S, or
wherein R¹ is 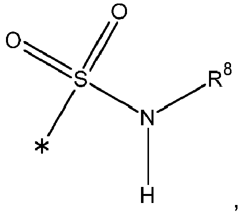 wherein R⁸ is hydroxy, and
wherein R² is selected from the group consisting of: 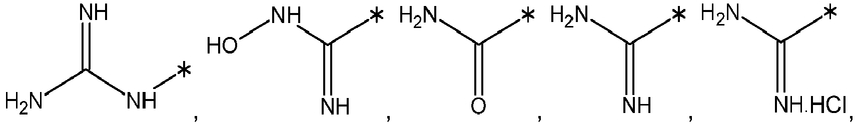 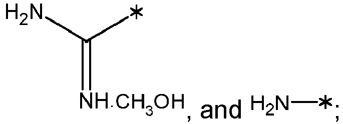 and
wherein * denotes the junction of the indicated bond with an atom of the compound of ***Formula A**;*

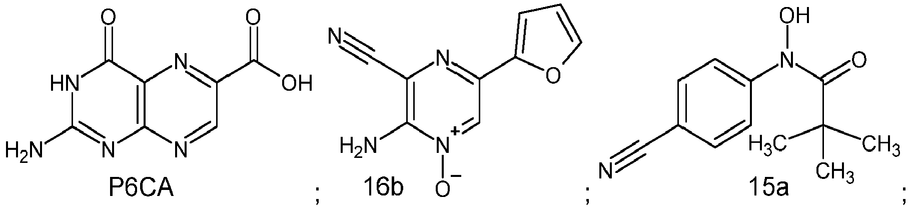

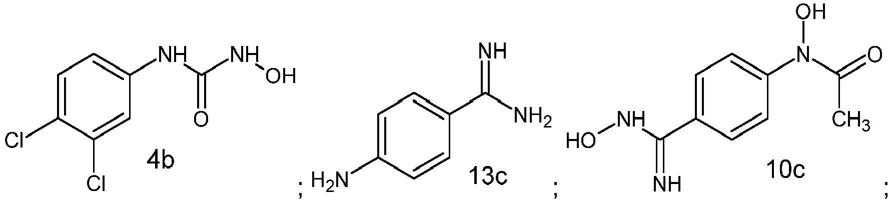

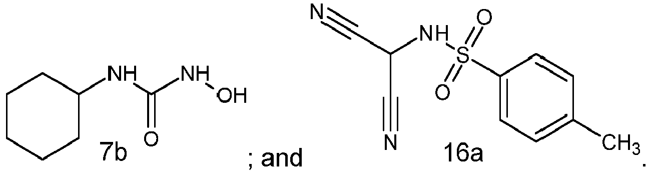

2. The compound of claim 1, wherein the compound of ***Formula A*** is:

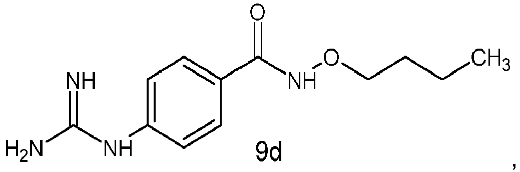

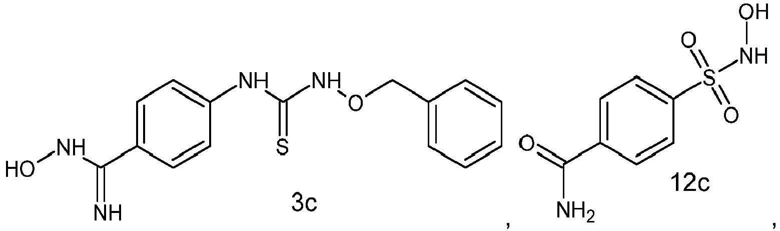

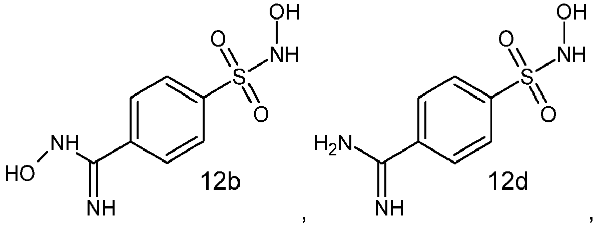

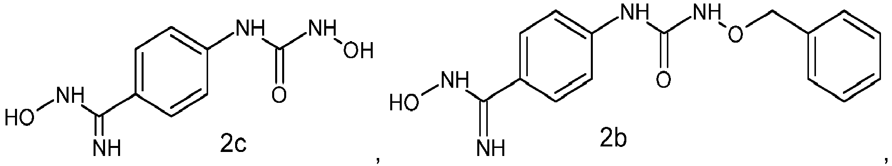

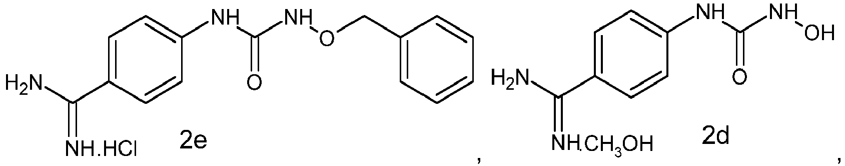

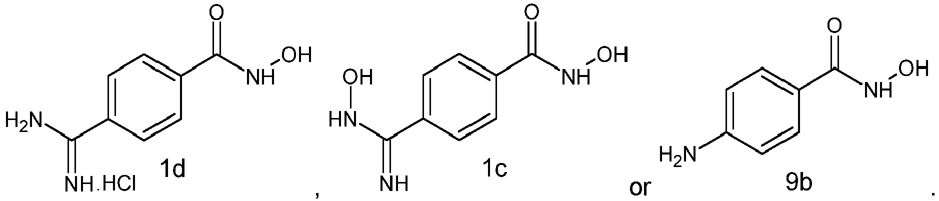

3. The compound of claim 2, wherein the compound of ***Formula A*** is:

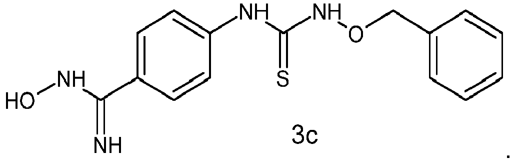

4. The compound of claim 2, wherein the compound of ***Formula A*** is:

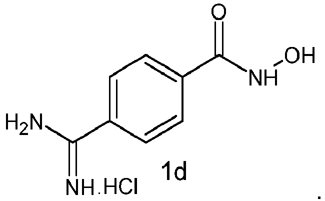

5. The compound of claim 2, wherein the compound of ***Formula A*** is:

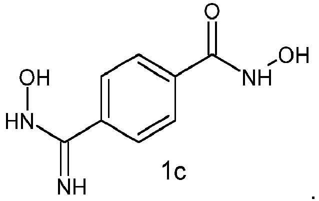

6. The compound of claim 2, wherein the compound of ***Formula A*** is:

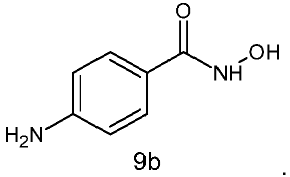

7. The compound according to any one of claims 1 to 6 for use as a medicament.

8. The compound according to any one of claims 1 to 6 for use in the treatment of an infectious disease.

9. The compound according to any one of claims 1 to 6 for use in the treatment of malaria.

10. Use of the compound according to any one of claims 1 to 6 for disrupting folate synthesis in *Plasmodium sp..*

11. The use of claim 10, wherein said *Plasmodium sp.* is *Plasmodium falciparum.*

12. Use of the compound according to any one of claims 1 to 6 for inhibiting 6-pyruvoyltetrahydropterin synthase (PTPS) in *Plasmodium sp..*

13. The use of claim 12, wherein said *Plasmodium sp.* is *Plasmodium falciparum* and said 6-pyruvoyltetrahydropterin synthase is *Plasmodium falciparum* 6-pyruvoyltetrahydropterin synthase (*PF*PTPS).

14. The use of claim 13, wherein said *Plasmodium falciparum* 6-pyruvoyltetrahydropterin synthase (*PF*PTPS) is PFF1360w.
